# EUROPEAN PATENT SPECIFICATION

(11) **EP 4 628 101 B1**
(45) Date of publication and mention of the grant of the patent: **04.11.2026**
(21) Application number: 25189316.0
(22) Date of filing: 18.09.2020
(51) Int. Cl.: A61K 47/06, A61K 35/64, A61K 35/06, A61K 31/5377, A61K 31/138, A61P 35/00, A61K 9/06, A61K 47/44, A61K 31/165

(54) **TRANSDERMAL PERMEATION ENHANCING COMPOSITION AND APPLICATION THEREOF IN TIMOLOL PREPARATION**
ZUSAMMENSETZUNG ZUR VERBESSERUNG DER TRANSDERMALEN PERMEATION UND ANWENDUNG DAVON IN DER TIMOLOLHERSTELLUNG
COMPOSITION D'AMÉLIORATION DE LA PERMÉATION TRANSDERMIQUE ET SON APPLICATION DANS UNE PRÉPARATION DE TIMOLOL

(30) Priority: 04.09.2019 CN 201910846541
(43) Date of publication of application: 08.10.2025
(62) Document number(s) of the earlier application(s) in accordance with Art. 76 EPC: 20860990.9 / 4 026 565
(73) Proprietor: Wuhan Conform Pharmaceutical Co., Ltd., Wuhan, Hubei 430074 (CN)
(72) Inventor: ZHOU, Xiaoshun, 430074 Wuhan (CN); MOU, Dongsheng, 430074 Wuhan (CN); TONG, Guofu, 430074 Wuhan (CN); LIU, Zhimei, 430074 Wuhan (CN); LIAO, Yuan, 430074 Wuhan (CN); HE, Rongli, 430074 Wuhan (CN); LIU, Wenshuang, 430074 Wuhan (CN)
(74) Representative: Osterhoff, Utz

(56) References cited:
- CN-A- 104 274 390
- CN-A- 105 106 105
- CN-A- 106 729 614
- US-A1- 2005 250 805

## Description

### TECHNICAL FIELD

The present invention relates to a composition formed mainly of a plurality of substances, which facilitates transdermal absorption of a drug.

### BACKGROUND

Infantile hemangiomas (IH) are one of the most common benign tumors in children worldwide. It was reported according to the literature that the incidence of hemangiomas in the general newborn population ranges from 1.1% to 2.6%, and up to 10% or more in premature infants, with the incidence in girls being three times higher than that in boys (Chinese Journal of Oral and Maxillofacial Surgery, 2011, 9(1): 61-67). Early literature reported that most infantile hemangiomas do not require treatment, but approximately 12% of high-risk patients may have to be treated due to impact on the organ function or even life-threatening conditions. Nowadays, in view of the potential harm of IH on the physiology and psychology of the child patient, especially the specific sites and types of IH, the treatment of hemangioma is suggested to follow the cosmetic principles: to pursue early intervention and treatment, and to choose the corresponding individualized treatment plan according to the site and progression of the disease, with the treatment aiming to restore normal skin color and texture (Chinese Journal of Oral and Maxillofacial Surgery, 2013, 11(2): 161-164; Shanghai Journal of Stomatology, 2016, 25(6): 744-747). There are now about 15 million newborns per year in China, based on which it is conservatively estimated that there are more than 200,000 new children with IH in China each year.

Hemangiomas are embryonic benign neoplastic malformations with the biological characteristics of proliferation and natural regression after proliferation of vascular endothelial cells, and the infantile hemangiomas are mainly clinically manifested in two completely distinct phases - a proliferative phase and a regressive phase. IH may not be apparent at birth, but enters the proliferative phase shortly after birth. The clinical manifestation is initially a small red plaque or papule, and the tumor continues to grow and gradually stabilize over the next 6 months to a year, usually showing histologically excessive division and proliferation of vascular endothelial cells, mast cell infiltration and thickening of the basement membrane layer. IH begins to enter a long period of regression when the child patient is about 1 year old, and this phase can last for several years or even more than ten years. The clinical manifestations are usually that the tumor growth stops gradually, the size continues to decrease, and the patient's skin begins to wrinkle and change from bright red to dark red. Like the proliferative phase, IH in the regressive phase also has varying degrees of mast cell infiltration and may still retain pigmentation, capillary dilation, fibrous and adipose tissue deposits and so on after completion of the regression. Strawberry hemangioma, part of cavernous hemangioma and part of mixed hemangioma in the traditional classification belong to this category, accounting for about 80% of congenital skin vascular disease. The etiology and pathogenesis of hemangiomas are not fully understood, but studies have shown that the pathogenesis is related to CD31, von Willebrand factor (vWF), vascular endothelial growth factor (VEGF), proliferating nuclear antigen, and urokinase that cause an imbalance between angiogenic and anti-angiogenic factors. In addition, endogenous corticosteroids and glucose transporter 1 (GLUT1) also play a role in the development of hemangiomas.

Since timolol was approved for the treatment of glaucoma in the United States in 1978, the safety of timolol as a first-line drug for the treatment of pediatric glaucoma has been verified in the pediatric population (Journal of Tissue Engineering and Reconstructive Surgery, 2012, 8(4): 208-212). Later, clinicians and researchers in various countries have successively reported that timolol is safe and effective in the treatment of IH. In view of the safety and efficacy of timolol, the American Association of Dermatologists and the American Association of Pediatricians have recommended topical timolol as one of the preferred options for the treatment of superficial localized infantile hemangiomas (smaller area) (J Pediatr Ophthalmol Strabismus, 2009, 46(1): 12-18). Clinical experts in China have also formed an expert consensus (Shanghai Journal of Stomatology, 2016, 25(6): 744-747) on the specific dosing method: 3 times a day, once every other 6-8 hours (once in the morning, once in the afternoon, and once in the evening), droping timolol eye drops (a concentration of 5mg/ml) on absorbent cotton or 1-2 layers of gauze (30-40µL/cm²) to be evenly soaked, applying the soaked absorbent cotton or gauze on the surface of the tumor, and keeping moist for 5-15min (depending on tumor thickness and treatment response).

Although timolol can be completely absorbed by the gastrointestinal tract, its hepatic first-pass effect and metabolism at other sites account for about 34% of the total dose, so delivery by gastrointestinal administration such as oral administration and other forms fails to achieve good bioavailability (J. Pharm. Pharmacol., 1978, 30, 53). Currently, there are two main types of timolol preparations on the market in China: timolol maleate eye drops and timolol maleate tablets. Among them, the strengths of the timolol maleate tablets are 5mg and 2.5mg, and the indications are: 1. essential hypertension, 2. treatment after angina pectoris or myocardial infarction, and 3. prevention of migraine. The strengths of the timolol maleate eye drops are 5ml:25mg, 5ml:12.5mg, 8ml:40mg, 10ml:50mg and 0.4ml:1mg, etc, and the indications are: reducing intraocular pressure in primary open-angle glaucoma. The effect of lowering intraocular pressure can be further enhanced for certain secondary glaucoma, ocular hypertension, some primary angle-closure glaucoma and other glaucoma that are ineffective by drugs and surgery.

Currently, topical treatment of IH with timolol is suitable for superficial types and small lesions. Hemangioma conditions located deep in the subcutis do not appear in the recommendations for topical treatment with timolol. Therefore, the transdermal effect of timolol in topical administration will directly affect the therapeutic effect.

Xu Yizhong et al. (Acta Pharmaceutica Sinica [J], 1992, 27(6): 467-471) studied the transdermal properties of timolol and the factors affecting its transdermal absorption (e.g., hydration, medium, pH, and various transdermal enhancers), and the skin stratum corneum affected the transdermal transport of timolol. Ji Xuefang et al. (Journal of China Pharmaceutical University [J], 1996, 27(1): 6-9) conducted a study on the effects of the selection, compatibility, ratio, action concentration and action site of the enhancers on the transdermal penetration of timolol maleate, and determined that a combination of laurocapam and propylene glycol could achieve the best transdermal effect.

China patent for invention No. 201410447231.7 discloses a long-acting transdermal preparation of timolol and application thereof in hemangioma, using menthol, sodium tetradecyl sulfate, geraniol, anethole or decyl methyl sulfoxide as a permeation enhancer, and combining with microneedles to achieve transdermal administration of timolol.

Further state of the art is provided by the documents US 2005/250805 A1, CN 105 106 105 A, CN 106 729 614 A and CN 104 274 390 A.

### SUMMARY

The object of the present invention is to provide a composition comprising timolol as an active ingredient, including a transdermal permeation enhancing composition that is applied to the skin surface to significantly promote the transdermal absorption of timolol.

The transdermal permeation enhancing composition consists of white vaseline, white beeswax and light liquid paraffin, wherein,
the amount of the white vaseline ranges from 68wt% to 93wt%, for example but not limited to, 68wt%, 69wt%, 70wt%, 71wt%, 72wt%, 73wt%, 74wt%, 75wt%, 76wt%, 77wt%, 78wt%, 79wt%, 80wt%, 81wt%, 82wt%, 83wt%, 84wt%, 85wt%, 86wt%, 87wt%, 88wt%, 89wt%, 90wt%, 91wt%, 92wt% and 93wt%;
the amount of the white beeswax ranges from 2wt% to 8wt%, for example but not limited to, 2wt%, 3wt%, 4wt%, 5wt%, 6wt%, 7wt% and 8wt%; and
the amount of the light liquid paraffin ranges from 5wt% to 30wt%, for example but not limited to, 5wt%, 6wt%, 7wt%, 8wt%, 9wt%, 10wt%, 11wt%, 12wt%, 13wt%, 14wt%, 15wt%, 16wt%, 17wt%, 18wt%, 19wt%, 20wt%, 21wt%, 22wt%, 23wt%, 24wt%, 25wt%, 26wt%, 27wt%, 28wt%, 29wt%, and 30wt%.

The transdermal permeation enhancing compositions of the present invention is used as transdermal permeation enhancers, and mixed with timolol to be applied to the skin surface, with the effect of promoting the transdermal absorption of timolol, wherein the concentration of timolol is 0.1wt %-3wt%.

One composition comprising timolol as an active ingredient uses a composition of white vaseline and wax substance as a transdermal permeation enhancer.

Another composition comprising timolol as an active ingredient further includes other beta blockers, such as but not limited to metoprolol and propranolol. These compounds are used in the present invention alone or in combination.

Other adjuvants include but are not limited to: lanolin, vegetable oil, sesame oil, peanut oil, cottonseed oil, dimethicone oil, glycerin, stearic acid, polyoxyethylene stearyl ether, octadecanol, cetearyl alcohol, polyoxyethylene stearate, and glycerol monostearate.

Another composition comprising timolol as an active ingredient contains 0.5wt% of timolol, 89.5wt% of white vaseline, 5wt% of white beeswax and 5wt% of light liquid paraffin.

Another composition comprising timolol as an active ingredient contains 0.5wt% of timolol, 84.5wt% of white vaseline, 5wt% of white beeswax and 10wt% of light liquid paraffin.

Another composition comprising timolol as an active ingredient contains 0.5wt% of timolol, 79.5wt% of white vaseline, 5wt% of white beeswax and 15wt% of light liquid paraffin.

Another composition comprising timolol as an active ingredient contains 0.5wt% of timolol, 69.5wt% of white vaseline, 5wt% of white beeswax and 25wt% of light liquid paraffin.

### DETAILED DESCRIPTION

The technical solutions of the present invention are described in detail hereinafter in connection with the accompanying drawings. The embodiments of the present invention are used only to illustrate the technical solutions of the invention and not to limit the invention.

The materials and methods used to verify the transdermal efficiency in the following embodiments of the present invention are specified as follows:

### 1. Preparation of ex vivo skin

Male piglets (age: 1 month) were taken, and their abdomen were dehaired with an electric shaver and the skin at the dehaired site was coated with a glycerin solution as a moisturizer. The piglets were sacrificed before the test. The hairless skin was taken, subcutaneous fat was removed, and the hairless skin with the subcutaneous fat removed was rinsed repeatedly with distilled water to be clean. The remaining distilled water on the skin surface was sucked up with filter paper, thereby obtaining the ex vivo skin.

### 2. Ex vivo transdermal device and method

A modified Franz vertical diffusion cell was used for a transdermal experiment. A stirrer was put into the receptor chamber. The piglet skin (piglet abdominal skin) was placed between the donor chamber and the receptor chamber, with the dermis facing the receptor solution, and fixed. 7mL of normal saline was added to the receptor chamber as the receptor solution. Then, about 0.5g of a test sample was applied to the piglet skin, and the effective permeation area was 3.14cm². The diffusion cell was placed in a constant temperature water bath (37°C±0.5°C), and a magnetic stirrer was placed under the constant temperature water bath with a stirring speed of 300rpm. Sampling 5mL of the receptor solution at 1h, 2h, 4h, 6h, 8h, 12h and 24h, while a same volume of a blank receptor solution was supplemented. The obtained samples were centrifuged. The supernatant was taken and the content of timolol maleate (or timolol) was determined by HPLC. $Q = \left[{C}_{n}\times v+{\sum }_{i = 1}^{n - 1} {C}_{i} \times 4\right] / S$

In the equation, Q denotes a cumulative penetration amount; S denotes an effective permeation area; V denotes a volume of normal saline in the receptor chamber; Ci denotes the concentration of drug in the receptor solution from the first to the last sampling; Cn denotes the concentration of drug in the receptor solution at the time of this sampling. The cumulative penetration amount (Q) per unit area was used to make a regression equation against time (t) to calculate the cumulative penetration amount per unit area.

### Example 1 Comparison of the transdermal effect of timolol maleate and timolol

### Preparation process of timolol maleate gel:

2% of hydroxypropyl methylcellulose and 15% of glycerol were weighed and mixed evenly, an appropriate amount of water was added, and the mixture was used as a gel matrix. 5% of timolol maleate was taken and dissolved completely with an appropriate amount of water (heated to 50°C and stirred) to obtain a timolol maleate solution. The dissolved timolol maleate solution was added to the gel matrix to obtain a mixture A. 2% of laurocapam and 5% of propylene glycol were weighed and mixed evenly to be added to the mixture A, and the obtained mixture was stirred well to obtain a timolol maleate gel sample.

### Preparation process of timolol ointment:

5% of white beeswax and 88% of white vaseline were weighed in a suitable container, heated in a water bath at 80°C to be melted, and mixed well while stirring to obtain a mixture A. After the temperature of the mixture A was slightly lowered, 5% of timolol and 2% of laurocapam were weighed and added into the mixture A to be dissolved completely by stirring. The heating device was removed, and the sample was continued to be stirred to cool the sample to room temperature, thereby obtaining a timolol ointment sample.

**Table 1 Drug cumulative penetration amount of the timolol maleate gel and timolol ointment (µg•cm⁻², n=5)**

| Formulation | | Time (h) | | | | | | |
|---|---|---|---|---|---|---|---|---|
| | | 1 | 2 | 4 | 6 | 8 | 12 | 24 |
| Timolol maleate gel (specification : 5%) | Abdome n | 0.18 | 0.37 | 0.80 | 1.67 | 2.86 | 8.12 | 131.70 |
| | | ±0.1 0 | ±0.2 3 | ±0.66 | ±1.47 | ±2.70 | ±9.15 | ±158.7 2 |
| Batch No.: 20160229 | | | | | | | | |
| Timolol Ointment (Specificatio n: 5%) | Abdome n | 0.68 | 6.60 | 70.60 | 168.8 4 | 271.1 4 | 453.0 7 | 1096.1 2 |
| | | ±0.4 4 | ±4.5 7 | ±23.6 4 | ±37.8 7 | ±45.4 2 | ±57.0 6 | ±77.77 |
| Batch No.: 20160302-1 | | | | | | | | |

By comparing the cumulative penetration amount results of the samples of timolol maleate and timolol preparations, it was found that the penetration rate of timolol in the timolol ointment was significantly higher than that of the timolol maleate gel preparation in abdominal skin of piglets, and the cumulative penetration amount of timolol in the ointment preparation was also significantly higher than that of timolol maleate in the gel preparation.

### Example 2 Comparison of transdermal effects of various timolol ointment formulation

As shown in Table 2, the prescribed amounts of light liquid paraffin, white vaseline and white beeswax were weighed in a suitable container in a water bath at 80°C until completely melted to obtain a mixture A. The mixture A was cooled to 65°C, and kept warm. The prescribed amount of timolol was weighed and added to the mixture A to be stirred for 6 minutes, so as to dissolve completely timolol, and the stirred mixture was continued to be stirred and cooled to room temperature, thereby obtaining the sample.

**Table 2 Various timolol ointment**

| Name | Formulation | | | | | |
|---|---|---|---|---|---|---|
| | 2016120 901 | 2016120 902 | 2016120 903 | 2016121 9-1 | 2016122 0-1 | 2016122 0-2 |
| Timolol | 0.5% | 0.5% | 0.5% | 0.5% | 0.5% | 0.5% |
| White vaseline | 99.5% | 94.5% | 94.5% | 89.5% | 79.5% | 69.5% |
| White beeswax | -- | 5% | -- | 5% | 5% | 5% |
| Light liquid paraffin | -- | -- | 5% | 5% | 15% | 25% |
| Total | 50g | 50g | 50g | 100g | 50g | 50g |

The above six samples were subjected to transdermal test to investigate the effect of different formulations on the transdermal behavior of the drug by in vitro transdermal test on the ex vivo abdominal skin of piglets. 4 mL of the receptor solution was taken out at 1 hour, 2 hours, 4 hours, 6 hours and 8 hours, respectively, while the same volume of the blank receptor solution was supplemented. The obtained samples were centrifuged, and the supernatant was taken and the concentration of timolol was determined by HPLC. The cumulative permeation amount (Q) per unit area was used to make a regression equation against time (t) to calculate the cumulative permeation amount per unit area (see Table 3 for details).

The displayed timolol ointments in Table 2 containing more than 93wt% white Vaseline are not part of the present invention.

**Table 3 Drug cumulative permeation amount of timolol in various ointment formulations (µg•cm⁻², n=10)**

| Formulation | Time (h) | | | | |
|---|---|---|---|---|---|
| | 1 | 2 | 4 | 6 | 8 |
| 2016120901 | 0.03 ±0.09 | 0.14 ±0.24 | 3.24 ±1.98 | 18.22 ±5.75 | 39.56 ±8.94 |
| 2016120902 | 0.11 ±0.28 | 0.47 ±0.87 | 8.00 ±5.25 | 30.30 ±10.30 | 56.02 ±13.58 |
| 2016120903 | 0.08 ±0.15 | 0.57 ±0.65 | 11.15 ±6.10 | 36.87 ±11.81 | 62.17 ±14.61 |
| 20161219-1 | 0.21 ±0.20 | 0.58 ±0.34 | 9.30 ±3.26 | 31.67 ±5.78 | 59.09 ±7.24 |
| 20161220-1 | 0.29 ±0.36 | 2.15 ±2.08 | 23.92 ±14.00 | 58.52 ±20.43 | 92.61 ±21.17 |
| 20161220-2 | 0.14 ±0.26 | 0.77 ±0.83 | 9.63 ±5.79 | 36.30 ±11.51 | 74.35 ±14.94 |

The results of the above transdermal tests indicate that:
the amount of timolol with the addition of light liquid paraffin or white beeswax alone or both light liquid paraffin and white beeswax in the formulations was improved compared with that of the ointment made with white vaseline alone.

The formulation No. 20161220-1 achieved the maximum transdermal effect for timolol, i.e., when the formulation contains 79.5 wt% white vaseline, 5 wt% white beeswax, and 15 wt% light liquid paraffin, the transdermal effect of timolol was better than those of the other formulations.

### Example 3 Comparison of the transdermal effect of various transdermal permeation enhancers on the timolol ointment formulation

The transdermal effect of the drug is directly related to the therapeutic effect, and the prior art also teaches that the use of a transdermal permeation enhancer such as propylene glycol, laurocapam, menthol, sodium tetradecyl sulfate, geraniol, anethole, and decyl methyl sulfoxide can improve the transdermal effect of timolol.

Based on the situation of application to infants, common transdermal penetration enhancers with low irritation properties, for example: terpenoids such as borneol; alcohols such as propylene glycol, and isopropyl myristate, are selected in this embodiment and their respective effects on the transdermal penetration of the drug was investigated.

Propylene glycol is generally considered non-toxic and low irritant and is commonly used in topical creams at a maximum dosage of 30%. Isopropyl myristate is widely used in cosmetic and topical preparations and is generally considered to be a non-toxic and non-irritating material. Borneol is a terpenoid compound, and generally less toxic than synthetic penetration enhancers, and relatively safe to use, and also have a good permeation promoting effect on hydrophilic and lipophilic drugs.

In addition, laurocapam is selected as a comparative study of penetration enhancers to investigate its effect on the transdermal effect of timolol.

As shown in Table 4, the prescribed amounts of light liquid paraffin, white vaseline and white beeswax were weighed in a suitable container in a water bath at 80°C until completely melted to obtain a mixture B. The mixture B was cooled to 65°C and kept warm. The prescribed amounts of timolol and the penetration enhancer were weighed and added into the mixture B to be stirred for 60 min to dissolve completely timolol and the penetration enhancer, and then the stirred mixture was continued to be stirred and cooled to room temperature, thereby obtaining the sample.

**Table 4 Timolol ointment with different penetration enhancers**

| Name | Formulation | | | |
|---|---|---|---|---|
| | 20170112-1 | 20170112-2 | 20170112-3 | 20170210 |
| Timolol | 0.5% | 0.5% | 0.5% | 0.5% |
| White vaseline | 77.5% | 77.5% | 77.5% | 77.5% |
| White beeswax | 5% | 5% | 5% | 5% |
| Light liquid paraffin | 15% | 15% | 15% | 15% |
| Propylene glycol | 2% | -- | -- | -- |
| Isopropyl myristate | -- | 2% | -- | -- |
| Laurocapam | -- | -- | -- | 2% |
| Borneol | -- | -- | 2% | -- |
| Total | 100g | 100g | 100g | 100g |

During the preparation, it was found that the mixture of propylene glycol and an oil based matrix such as white vaseline were not miscible and propylene glycol was not uniformly distributed in the mixture matrix.

The samples with the addition of penetration enhancers in Table 4 above were subjected to transdermal test, and the effect of different penetration enhancers on the drug transdermal behavior was investigated by in vitro transdermal test on the ex vivo abdominal skin of piglets. 4mL of the receptor solution was taken out at 1h, 2h, 4h, 6h and 8h, respectively, while the same volume of the blank receptor solution was supplemented. The obtained samples were centrifuged, and the supernatant was taken and the concentration of timolol was determined by HPLC. The cumulative penetration amount (Q) per unit area was used to make a regression equation against time (t) to calculate the cumulative penetration amount, and the results of the transdermal test were shown in Table 5 below.

**Table 5 Drug cumulative penetration amount of timolol ointment containing different penetration enhancers (µg•cm⁻², n=10)**

| Formulation | Time (h) | | | | |
|---|---|---|---|---|---|
| | 1 | 2 | 4 | 6 | 8 |
| 20161220-1 | 0.29 | 2.15 | 23.92 | 58.52 | 92.61 |
| | ±0.36 | ±2.08 | ±14.00 | ±20.43 | ±21.17 |
| 20170112-1 | 0.00 | 0.07 | 3.45 | 17.38 | 36.12 |
| | ±0.00 | ±0.14 | ±1.75 | ±6.21 | ±8.89 |
| 20170112-3 | 0.23 | 0.46 | 1.33 | 4.94 | 13.42 |
| | ±0.39 | ±0.65 | ±1.51 | ±4.23 | ±8.46 |
| 20170112-2 | 0.00 | 0.04 | 1.65 | 10.47 | 30.23 |
| | ±0.00 | ±0.14 | ±1.25 | ±5.88 | ±11.61 |
| 20170210 | 0.19 | 0.70 | 2.81 | 9.30 | 20.61 |
| | ±0.50 | ±1.70 | ±4.56 | ±8.71 | ±12.40 |

The results of the transdermal test showed that the penetration amount of timolol in the four samples with the separate addition of propylene glycol, borneol, isopropyl myristate and laurocapam as transdermal penetration enhancers was significantly lower than that of the ointment sample without the addition of any penetration enhancer, indicating that the addition of various existing penetration enhancers not only failed to enhance the transdermal effect of the drug in the timolol ointment formulations of this embodiment, but also reduced the drug permeation amount. Compared with timolol maleate eye drops, the transdermal effect of the ointment product of this embodiment without the addition of any penetration enhancer has been significantly improved (see Table 7 for details on cumulative penetration amount), while the addition of penetration enhancers may cause safety issues such as skin irritation, especially when the ointment product of this embodiment is used in the special population like infants who have more sensitive and fragile skin. Therefore, it has been verified that the ointment product of this embodiment does not require the addition of any of the currently common penetration enhancers in the case of a penetration enhancer consisting of white vaseline and wax substance.

Additional tests have shown that the transdermal results of the timolol ointment (i.e., using a penetration enhancer consisting of white vaseline and wax substance) of this embodiment versus timolol maleate eye drops on piglets indicate that the blood drug concentrations of timolol after dermally apply the timolol ointment are significantly higher than the timolol maleate eye drops.

### Example 4 Study of dosage accuracy during administration

The administered dose will affect the safety and efficacy of the medication. In order to accurately control the administered dose, the ointment was packaged in a tube with a quantitative pump device in this embodiment. Theoretically, the quantitative pump pumped a fixed amount of medicine every time when it is pressed, but the viscosity and hardness of the sample inside the tube may affect the accuracy of the pumped amount. Based on the analysis of the results in the stability study, the errors of pumping out the samples containing 5% of light liquid paraffin and 10% of light liquid paraffin were investigated respectively (see Table 6 for details). The formulation with good pumped amount consistency were further selected by comparing the RSD of 10 consecutive pumped weights.

Determination method: according to the instruction of the sample tube with a quantitative pump, a cap was removed, and the quantitative pump was pressed continuously until the sample was squeezed out. The samples at the first 15 pumps were discarded (to reduce the error caused by the possible instability of the sample volume when the sample was just squeezed out), and then the amounts of 10 consecutive pumps were measured, 5 tubes for each formulation.

**Table 6 Examination of the pumped amount of timolol ointment in different formulations**

| Formulation | Examinati on items | Sample | | | | |
|---|---|---|---|---|---|---|
| | | 1 | 2 | 3 | 4 | 5 |
| 0.5% timolol ointment (5% of white beeswax + 5% of light liquid paraffin) | Mean weight (M) | 0.100 | 0.099 | 0.103 | 0.111 | 0.099 |
| | RSD (%) | 2.32 | 3.02 | 2.64 | 1.64 | 3.73 |
| 0.5% timolol ointment (5% of white beeswax + 10% of light liquid paraffin) | Mean weight (M) | 0.100 | 0.101 | 0.099 | 0.094 | 0.103 |
| | RSD (%) | 1.29 | 1.51 | 0.098 | 1.1 | 1.41 |

The results showed that the samples were squeezed out at about 0.1 g per pump, the error in the pumped amount was smaller when the formulation contained 10% of light liquid paraffin, and the squeezing pressure used was smaller and the samples were relatively easy to pump out. With 5% of light liquid paraffin, a larger squeezing pressure was required to pump out the sample, and the error was somewhat larger compared with the sample which contain 10% of the light liquid paraffin.

Therefore, the formulation containing 0.5 wt% of timolol, 84.5 wt% of white vaseline, 5 wt% of white beeswax, and 10 wt% of light liquid paraffin is more suitable for making ointment preparations.

The effect of a transdermal penetration enhancer on the transdermal effect of the timolol ointment was examined to help us understand whether the addition of the transdermal penetration enhancer was necessary.

### Example 5 Comparison of cumulative permeation amount, intradermal retention and supra-dermal residues

The results of timolol ointment (containing 0.5 wt% timolol, 84.5 wt% white vaseline, 5 wt% white beeswax and 10 wt% light liquid paraffin) and timolol maleate eye drops (commercially available, Wuhan Wujing Pharmaceutical Co., Ltd.) were compared in terms of cumulative penetration amount, intradermal retention and supra-dermal residues, as shown in Table 7.

**Table 7 Drug cumulative permeation amount, intradermal retention and supra-dermal residual amount**

| Sam ple | Cumulative permeation amount (µg•cm⁻²) | | | | | Subcutaneous | | Inter-dermal | | Supra-dermal | |
|---|---|---|---|---|---|---|---|---|---|---|---|
| | 1(h ) | 2(h ) | 4(h ) | 6(h) | 8(h) | Cumul ative perme ation amoun t (µg) | Cumul ative perme ation rate (%) | Rete ntion amou nt (µg) | Rete ntion rate (%) | Resi dual amo unt (µg) | Resi dual rate (%) |
| Timo lol oint ment (n=8) | 0.0 9 ±0. 24 | 0.7 3 ±1. 30 | 11. 44 ±7. 03 | 34. 31 ±10 .50 | 59. 25 ±10 .61 | 134.50 | 6.55 | 100.4 9 | 4.90 | 1701 .25 | 82.9 2 |
| Timo lol male ate eye drop s (n=4) | 0.0 0 ±0. 00 | 0.0 0 ±0. 00 | 0.2 7 ±0. 25 | 0.7 3 ±0. 66 | 1.6 5 ±1. 13 | 3.39 | 0.17 | 96.67 | 4.69 | 1738 .37 | 84.3 9 |

It was shown in Table 7 that although the drug cumulative permeation amount of the timolol ointment was significantly higher than that of the timolol maleate eye drops, the intradermal retention amount of the drug on the skin was consistent, indicating that the timolol ointment does not retain a significant amount in the epidermal tissue of the skin and cause adverse skin reactions (it has been reported that the timolol maleate eye drops may cause adverse reactions such as skin desquamation and itching when applied topically).

### Example 6 Skin irritation

Prescribed amounts of light liquid paraffin, white beeswax and white vaseline, as shown in Table 8, were weighed in a dry beaker, heated in a water bath at 80°C to be melted, and mixed well by stirring for about 30 min. After that, the mixture was stirred and cooled to room temperature and filled, thereby obtaining the blank samples. The prepared blank ointment samples with different formulations was numbered 1, 2, 3 and 4, respectively. 30 voluntary subjects were selected, and each sample was squeezed out approximately 0.5g and applied to the forearm . After the sample was applied evenly and left for approximately 20 minutes, the skin reaction at the site of ointment application was observed and scored according to the reaction of redness and swelling, tingling and itching for evaluation.

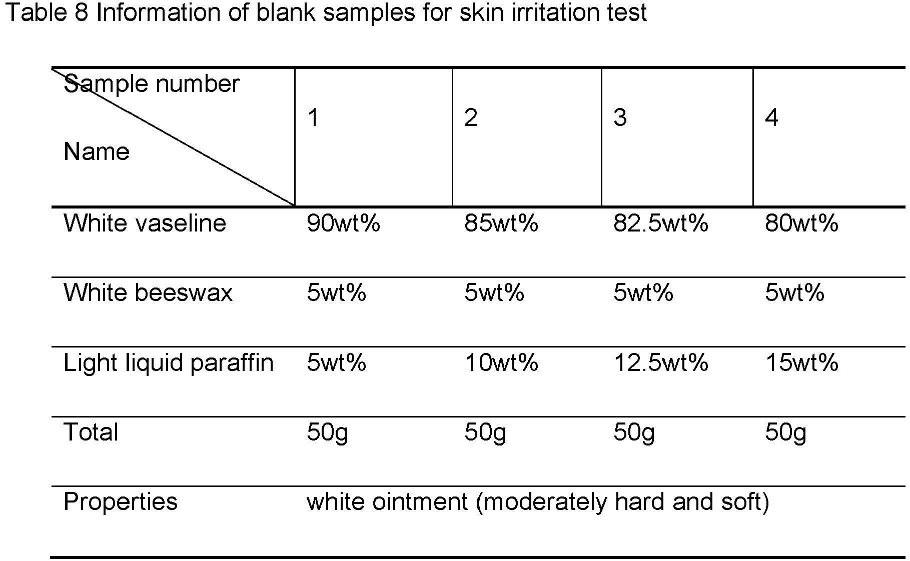

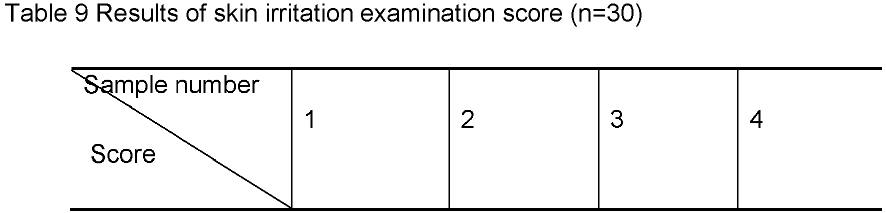

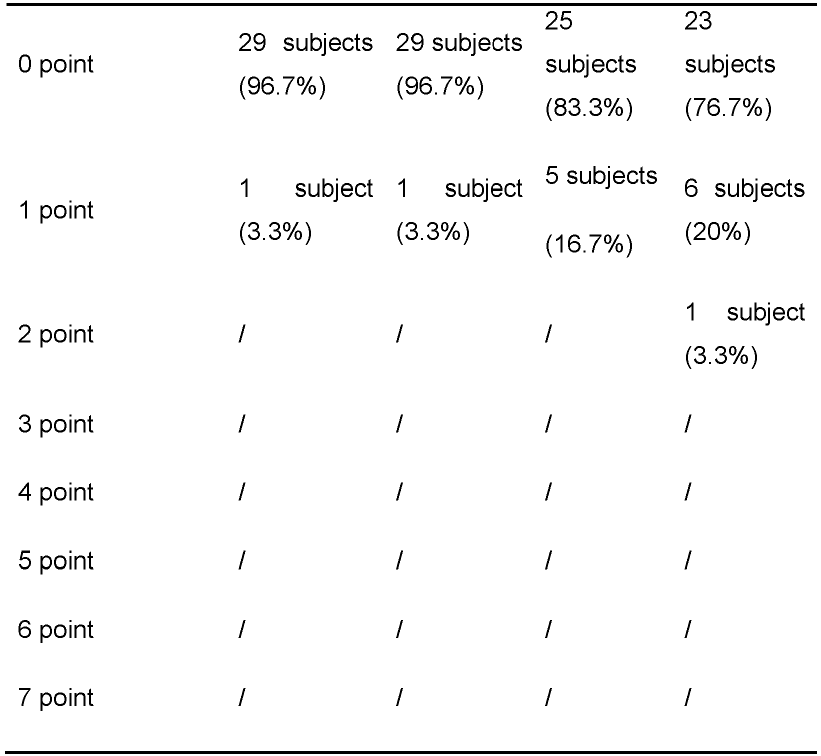

The skin irritation and/or allergic reaction scores listed by the FDA are based on the methodology used by Hill Top Research. Specific skin reaction scoring criteria is:
0 point: no irritation seen;
1 point: slight erythema, just observable;
2 points: significant erythema, easily visible to the naked eye, slight edema or slight papular reaction;
3 points: erythema and papules;
4 points: significant edema;
5 points: erythema, edema and papules;
6 points: blisters; and
7 points: strong reactions with a distribution range beyond the test site.

The results of the skin irritation scores of the blank ointments No. 1 to 4 in Table 8 are shown in Table 9.

The results of the skin irritation test indicated that:
Overall, no significant adverse skin reactions (scores of 3 points and more) were observed for all samples over the short test period, which initially indicated that the safety of the matrix was relatively good.

There was a trend towards an increase in the number of the subjects with slight skin reactions as the light liquid paraffin content increased, suggesting that an increase in the light liquid paraffin content may cause a lesser amount of skin irritation (or allergic reactions), which is consistent with the information known about the nature of the adjuvants.

In addition, those voluntary subjects with mild skin reactions, after the self control, generally reflected that samples 3 and 4 with the light liquid paraffin content higher than 10% caused a slight burning sensation on the skin when used, while the samples with the light liquid paraffin content of 10wt% and 5% had no any skin irritation, and the same voluntary subject showed very slight skin reactions in samples 1 and 2. Thus, the preliminary judgment is that the skin safety is higher when the concentration of the light liquid paraffin in the timolol ointment is not higher than 10wt%.

## Claims

1. A composition comprising timolol as an active ingredient and a transdermal permeation enhancing composition for use in the treatment of infantile hemangiomas, wherein the transdermal permeation enhancing composition consists of white Vaseline, a light liquid paraffin and white beeswax, wherein an amount of the white Vaseline is from 68wt% to 93wt%, an amount of the light liquid paraffin is from 5wt% to 30wt%, and the amount of the white beeswax is from 2wt% to 8wt%, wherein a concentration of timolol is 0.1wt% to 3wt%.

2. The composition for use according to claim 1, further comprising one or more of metoprolol and propranolol.

3. The composition for use according to claim 1, wherein the composition is selected from one of following combinations:
0.5 wt% of timolol, 89.5 wt% of white Vaseline, 5 wt% of white beeswax and 5 wt% of light liquid paraffin;
0.5 wt% of timolol, 84.5 wt% of white Vaseline, 5 wt% of white beeswax and 10 wt% of light liquid paraffin;
0.5 wt% of timolol, 79.5 wt% of white Vaseline, 5 wt% of white beeswax and 15 wt% of light liquid paraffin; and
0.5 wt% of timolol, 69.5 wt% of white Vaseline, 5 wt% of white beeswax and 25 wt% of light liquid paraffin.

## Patentansprüche

1. Zusammensetzung, umfassend Timolol als Wirkstoff und eine die transdermale Permeation verstärkende Zusammensetzung, zur Verwendung bei der Behandlung infantiler Hämangiome, wobei die die transdermale Permeation verstärkende Zusammensetzung aus weißer Vaseline, dünnflüssigem Paraffin und weißem Bienenwachs besteht, wobei der Anteil der weißen Vaseline 68 Gew.-% bis 93 Gew.-%, der Anteil des dünnflüssigen Paraffins 5 Gew.-% bis 30 Gew.-% und der Anteil des weißen Bienenwachses 2 Gew.-% bis 8 Gew.-% beträgt und wobei die Konzentration von Timolol 0,1 Gew.-% bis 3 Gew.-% beträgt.

2. Zusammensetzung zur Verwendung nach Anspruch 1, ferner umfassend mindestens einen Wirkstoff ausgewählt aus Metoprolol und Propranolol.

3. Zusammensetzung zur Verwendung nach Anspruch 1, wobei die Zusammensetzung aus einer der folgenden Kombinationen ausgewählt ist:
0,5 Gew.-% Timolol, 89,5 Gew.-% weiße Vaseline, 5 Gew.-% weißes Bienenwachs und 5 Gew.-% dünnflüssiges Paraffin;
0,5 Gew.-% Timolol, 84,5 Gew.-% weiße Vaseline, 5 Gew.-% weißes Bienenwachs und 10 Gew.-% dünnflüssiges Paraffin;
0,5 Gew.-% Timolol, 79,5 Gew.-% weiße Vaseline, 5 Gew.-% weißes Bienenwachs und 15 Gew.-% dünnflüssiges Paraffin; und
0,5 Gew.-% Timolol, 69,5 Gew.-% weiße Vaseline, 5 Gew.-% weißes Bienenwachs und 25 Gew.-% dünnflüssiges Paraffin.

## Revendications

1. Composition comprenant du timolol en tant que principe actif et une composition favorisant la perméation transdermique, pour son utilisation dans le traitement des hémangiomes infantiles, dans laquelle la composition favorisant la perméation transdermique est constituée de vaseline blanche, de paraffine liquide légère et de cire d'abeille blanche, dans laquelle la quantité de vaseline blanche est comprise entre 68 % en poids et 93 % en poids, la quantité de paraffine liquide légère est comprise entre 5 % en poids et 30 % en poids et la quantité de cire d'abeille blanche est comprise entre 2 % en poids et 8 % en poids, et dans laquelle la concentration de timolol est comprise entre 0,1 % en poids et 3 % en poids.

2. Composition pour son utilisation selon la revendication 1, comprenant en outre au moins un principe actif choisi parmi le métoprolol et le propranolol.

3. Composition pour son utilisation selon la revendication 1, dans laquelle la composition est choisie parmi l'une des combinaisons suivantes:
0,5 % en poids de timolol, 89,5 % en poids de vaseline blanche, 5 % en poids de cire d'abeille blanche et 5 % en poids de paraffine liquide légère;
0,5 % en poids de timolol, 84,5 % en poids de vaseline blanche, 5 % en poids de cire d'abeille blanche et 10 % en poids de paraffine liquide légère;
0,5 % en poids de timolol, 79,5 % en poids de vaseline blanche, 5 % en poids de cire d'abeille blanche et 15 % en poids de paraffine liquide légère et
0,5 % en poids de timolol, 69,5 % en poids de vaseline blanche, 5 % en poids de cire d'abeille blanche et 25 % en poids de paraffine liquide légère.
